# EUROPÄISCHE PATENTSCHRIFT

(11) **EP 0 696 288 B1**
(45) Veröffentlichungstag und Bekanntmachung des Hinweises auf die Patenterteilung: **20.09.2000**
(21) Anmeldenummer: 94914313.5
(22) Anmeldetag: 28.04.1994
(51) Int. Cl.: C07F 9/6509, C07F 9/6558, A61K 31/66

(54) **NEUE CHINOXALINDIONDERIVATIVE, DEREN HERSTELLUNG UND VERWENDUNG IN ARZNEIMITTELN**
QUINOXALINDIONE DERIVATIVES, THEIR PREPARATION AND THEIR USE IN DRUGS
NOUVEAUX DERIVES DE QUINOXALINEDIONE, LEUR FABRICATION ET LEUR UTILISATION DANS DES MEDICAMENTS

(30) Priorität: 28.04.1993 DE 4314591; 21.12.1993 DE 4344486
(43) Veröffentlichungstag der Anmeldung: 14.02.1996
(60) Teilanmeldung: 00250046.0
(73) Patentinhaber: SCHERING AKTIENGESELLSCHAFT, 13353 Berlin (DE)
(72) Erfinder: HUTH, Andreas, D-12437 Berlin (DE); TURSKI, Lechoslaw, D-13505 Berlin (DE)
(86) Internationale Anmeldenummer: DE9400493
(87) Internationale Veröffentlichungsnummer: WO9425469

(56) Entgegenhaltungen:
- EP-A- 0 315 959
- EP-A- 0 344 943
- EP-A- 0 556 393
- WO-A-91/13878
- WO-A-93/08173
- WO-A-94/00124

## Beschreibung

Die Erfindung betrifft Chinoxalindionderivate, deren Herstellung und Verwendung in Arzneimitteln.

Es ist bekannt, daß Chinoxalinderivate Affinität an die Quisqualat-Rezeptoren besitzen und sich auf Grund der Affinität als Arzneimittel zur Behandlung von Krankheiten des zentralen Nervensystems eignen. So sind ähnlich substituierte Chinoxaline aus EP-A-344 943, WO 91/13 878, EP-A-315 959, WO 92/07847 und WO 94/00124 bekannt.

Die erfindungsgemäßen Verbindungen haben die Formel I 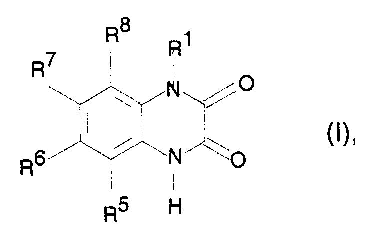 worin
R¹ -(CH₂)ₙ-CR²H-(CH₂)ₘ-Z und
R⁵, R⁶, R⁷ und R⁸ gleich oder verschieden sind und Wasserstoff C₁₋₆-Alkyl, CF₃, Nitro, Halogen, NR⁹R¹⁰, Cyano, SOₚR¹¹, SO₂NR¹²R¹³, SO₃H, SO₃C₁₋₆-Alkyl oder OR¹⁴ bedeuten,
   wobei
R² Wasserstoff oder -(CH₂)_{q}-R³,
R³ Wasserstoff, Hydroxy, C₁₋₆-Alkoxy oder NR¹⁵R¹⁶,
n, m und q jeweils 0, 1, 2 oder 3
Z POXY oder OPOXY,
R¹¹ H, C₁₋₆-Alkyl, Phenyl,
p 0, 1 oder 2
R¹², R¹³ Wasserstoff oder C₁₋₄-Alkyl,
R¹⁴ H oder gegebenenfalls 1-3 fach mit Halogen substituiertes C₁₋₆-Alkyl,
X und Y gleich oder verschieden sind und Hydroxy, C₁₋₆-Alkoxy, C₁₋₄-Alkyl oder NR¹⁸R¹⁹ bedeuten,
R⁹ und R¹⁰ gleich oder verschieden sind und Wasserstoff, Phenyl oder C₁₋₆-Alkyl, das gegebenenfalls mit C₁₋₄-Alkoxy oder einer gegebenenfalls mit C₁₋₄-Alkyl mono- oder di-substituierter Aminogruppe substituiert sein kann, oder gemeinsam mit dem Stickstoffatom einen 5 - 7gliedrigen gesättigten Heterocyclus bilden, der ein weiteres N-, S- oder O-Atom enthalten und substituiert sein kann oder einen ungesättigten 5gliedrigen Heterocyclus bilden, der 1-3 N-Atome enthalten und substituiert sein kann,
R¹⁵ und R¹⁶, R¹⁸ und R¹⁹ gleich oder verschieden sind und Wasserstoff, C₁₋₄-Alkyl, Phenyl oder gemeinsam mit dem Stickstoffatom einen 5 - 7gliedrigen gesättigten Heterocyclus bilden, der ein weiteres Sauerstoff-, Schwefel- oder Stickstoffatom enthalten und substituiert sein kann oder einen ungesättigten 5gliedrigen Heterocyclus bilden, der 1-3 N-Atome enthalten und substituiert sein kann,
sowie deren Isomeren und Salze, die in Position 5 - 8 zweifach substituiert sind, und
falls R¹ Methanphosphonsäure und R⁶ CF₃ oder NO₂ und R⁷ Imidazol ist, können R⁵ und R⁸ nicht gleichzeitig Wasserstoff sein.

Die Verbindungen der allgemeinen Formel I beinhalten auch die möglichen tautomeren Formen und umfassen die E- oder Z-Isomeren oder, falls ein chirales Zentrum vorhanden ist, die Razemate oder Enantiomeren.

Die Substituenten stehen bevorzugt in 6- und/oder 7-Stellung.

Unter Alkyl ist jeweils ein geradkettiger oder verzweigter Alkylrest zu verstehen wie beispielsweise Methyl, Ethyl, Propyl, Isopropyl, Butyl, Isobutyl, sek. Butyl, Pentyl, Isopentyl, Hexyl, wobei C₁₋₄-Alkylreste bevorzugt werden.

Unter Halogen ist jeweils Fluor, Chlor, Brom und Jod, insbesondere Fluor, Chlor und Brom, zu verstehen.

Bilden R⁹ und R¹⁰, R¹⁵ und R¹⁶, R¹⁸ und R¹⁹ gemeinsam mit dem Stickstoffatom einen gesättigten Heterocyclus, so ist beispielsweise Piperidin, Pyrrolidin, Morpholin, Thiomorpholin, Hexahydroazepin oder Piperazin gemeint. Der Heterocyclus kann 1 - 3fach substituiert sein mit C₁₋₄-Alkyl oder Aryl wie Phenyl. Beispielsweise seien genannt N-Methyl-piperazin, 2,6-Dimethylmorpholin, oder Phenylpiperazin.

Bilden R⁹ und R¹⁰, R¹⁵ und R¹⁶, R¹⁸ und R¹⁹, gemeinsam mit dem Stickstoffatom einen ungesättigten Heterocyclus, so seien beispielsweise Imidazol, Pyrazol, Pyrrol und Triazol genannt, die ein- bis zweifach mit Cyano, C₁₋₄-Alkyl, Phenyl oder CO₂C₁₋₆-Alkyl substituiert sein können.

Ist eine saure Funktion enthalten, sind als Salze die physiologisch verträglichen Salze organischer und anorganischer Basen geeignet wie beispielsweise die gut löslichen Alkali- und Erdalkalisalze sowie N-Methyl-glukamin, Dimethyl-glukamin, Ethylglukamin, Lysin, 1,6-Hexandiamin, Ethanolamin, Glukosamin, Sarkosin, Serinol, Trishydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie HCl, H₂SO₄, Phosphorsäure, Zitronensäure, Weinsäure u.a.

Bevorzugt sind Verbindungen, die in 5-, 6-, 7- und/oder 8-Stellung mit C₁₋₆-Alkyl, CF₃, Nitro, Halogen, SOₚR¹¹, SO₂NR¹²R¹³ oder NR⁹R¹⁰ substituiert sind.

Besonders bevorzugt sind Phosphonsäurederivate, die ein- bis zweifach substituiert sein können. Insbesondere bevorzugt sind Phosphonsäurederivate, die in Position 5 - 8 zweifach substituiert sind. Bevorzugte Substituenten R⁵ - R⁸ sind insbesondere NR⁹R¹⁰ und CF₃.

Die Verbindungen der Formel I sowie deren physiologisch verträglichen Salze sind auf Grund ihrer Affinität zu den AMPA-Rezeptoren als Arzneimittel verwendbar. Auf Grund ihres Wirkprofils eignen sich die erfindungsgemäßen Verbindungen zur Behandlung von Krankheiten, die durch Hyperaktivität excitatorischer Aminosäuren wie zum Beispiel Glutamat oder Aspartat hervorgerufen werden. Da die neuen Verbindungen als Antagonisten excitatorischer Aminosäuren wirken und eine hohe spezifische Affinität zu den AMPA-Rezeptoren zeigen, indem sie den radioaktiv markierten spezifischen Agonisten (RS)α-Amino-3-hydroxy-5-methyl-4-isoxazolpropionat (AMPA) von den AMPA-Rezeptoren verdrängen, eignen sie sich insbesondere zur Behandlung von solchen Krankheiten, die über die Rezeptoren excitatorischer Aminosäuren, insbesondere den AMPA-Rezeptor, beeinflußt werden.

Erfindungsgemäß können die Verbindungen verwendet werden zur Behandlung neurologischer und psychiatrischer Störungen, die durch die Überstimulation des AMPA-Rezeptors ausgelöst werden. Zu den neurologischen Erkrankungen , die funktionell und präventiv behandelt werden können, gehören zum Beispiel neurodegenerative Störungen wie Morbus Parkinson, Morbus Alzheimer, Chorea Huntington, Amyotrophe Lateralsklerose und Olivopontocerebelläre Degeneration. Erfindungsgemäß können die Verbindungen zur Prävention des postischämischen Zelluntergangs, des Zelluntergangs nach Hirntrauma, bei Schlaganfall, Hypoxie, Anoxie und Hypoglykämie und zur Behandlung der Senilen Demenz, Multiinfarkt Demenz sowie Epilepsie und Muskelspastik verwendet werden. Zu den psychiatrischen Erkrankungen gehören Angstzustände, Schizophrenie, Migräne, Schmerzzustände, sowie die Behandlung von Schlafstörungen und der Entzugssymptomatik nach Drogenmißbrauch wie bei Alkohol-, Kokain-, Benzodiazepin- oder Opiat-Entzug.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5 - 1000 mg, vorzugsweise 50 - 200 mg, wobei die Dosis als einmal zu verabreichende Einzeldosis oder unterteilt in 2 oder mehreren Tagesdosen gegeben werden kann.

Die Herstellung der erfindungsgemäßen Verbindungen erfolgt nach an sich bekannten Methoden. Beispielsweise gelangt man zu Verbindungen der Formel I dadurch, daß man
a) eine Verbindung der Formel II 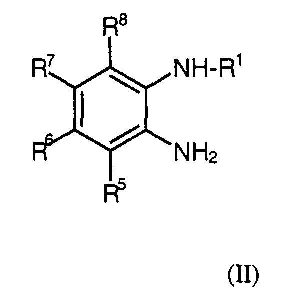 worin R¹ bis R⁸ die obige Bedeutung haben, mit Oxalsäure oder reaktiven Oxalsäurederivaten cyclisiert oder
b) eine Verbindung der Formel III 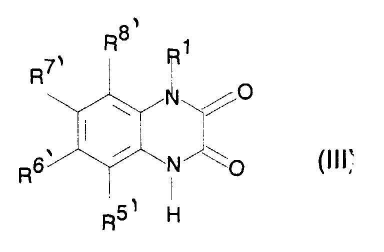 worin R¹ die obige Bedeutung hat und einer der Substituenten R^{5'}, R^{6'}, R^{7'} oder R^{8'} eine Fluchtgruppe darstellt, nucleophil substituiert und gewünschtenfalls anschließend die Estergruppe verseift oder die Säuregruppe verestert oder amidiert oder die Nitrogruppe reduziert zur Aminogruppe oder die Aminogruppe alkyliert oder acyliert oder die Aminogruppe gegen Halogen oder Cyano austauscht oder eine Nitrogruppe oder Halogen einführt oder eine Etherspaltung vornimmt oder den Alkohol in Halogenid überführt oder dieses Halogen nukleophil substituiert oder ein Nitril in das Tetrazol überführt oder die Isomeren trennt oder die Salze bildet.

Die Cyclisierung zu Verbindungen der Formel I erfolgt mit Oxalsäure in bekannter Weise einstufig in saurem Milieu oder mit einem reaktiven Oxalsäurederivat einstufig oder auch zweistufig. Als bevorzugt ist das Zweistufenverfahren zu betrachten, bei dem das Diamin mit einem Oxalsäurederivat wie dem Oxalesterhalbchlorid oder reaktiven Oxalsäureimidazolidderivaten in polaren Lösungsmitteln wie cyclischen oder acyclischen Ethern oder halogenierten Kohlenwasserstoffen beispielsweise Tetrahydrofuran, Diethylether oder Methylenchlorid in Gegenwart einer Base wie organischen Aminen beispielsweise Triethylamin, Pyridin, Hünig-Base oder Dimethylaminopyridin umgesetzt wird. Die anschließende Cyclisierung kann basisch oder auch sauer, vorzugsweise aber in saurem Milieu durchgeführt werden, wobei der Reaktionsmischung Alkohol als Lösungsvermittler zugesetzt werden kann.

Geeignete Basen für das Zweistufenverfahren stellen auch Alkalihydride dar wie NaH, die in inerten Lösungsmitteln wie Kohlenwasserstoffen oder Ethern eingesetzt werden.

Als Fluchtgruppen in der Verfahrensvariante b) sowie bei der Herstellung der Ausgangsverbindungen der Formel II sind Halogene wie Fluor, Chlor, Brom, Jod oder O-Mesylat, O-Tosylat, O-Triflat oder O-Nonaflat geeignet. Die nucleophile Substitution wird nach literaturbekannten Methoden in Gegenwart einer Base durchgeführt und wird durch eine aktivierende elektronenziehende Gruppe wie z.B. Nitro, Cyano, Trifluormethyl vorzugsweise in o-Stellung begünstigt.

Als Nucleophile sind beispielsweise primäre und sekundäre Amine, N-enthaltende ungesättigte oder gesättigte Heterocyclen, Cyanid, Alkoholate, Thiolate u.a. geeignet. Die Umsetzung kann in polaren Lösungsmitteln wie Alkoholen, halogenierten Kohlenwasserstoffen, Dimethylacetamid, Acetonitril oder Wasser oder ohne Lösungsmittel vorgenommen werden. Als Basen sind anorganische Basen wie Alkali- oder Erdalkalihydroxide oder -carbonate oder organische Basen wie cyclische, alicyclische und aromatische Amine, wie DBU, Hünigbase, Pyridin oder Dimethylaminopyridin geeignet.

Als Base kann im Fall von Aminen das Nucleophil selbst im Überschuß benutzt werden, wobei man gegebenenfalls ohne weiteres Lösungsmittel arbeiten kann. Bei zu niedrigem Siedepunkt des Amins kann gegebenenfalls unter Druck im Autoklaven gearbeitet werden.

Die sich gegebenenfalls anschließende Verseifung einer Estergruppe kann basisch oder vorzugsweise sauer erfolgen, indem man bei erhöhter Temperatur bis zur Siedetemperatur des Reaktionsgemisches in Gegenwart von Säuren wie hoch konzentrierter wäßriger Salzsäure gegebenenfalls in Lösungsmitteln wie beispielsweise Trifluoressigsäure oder Alkoholen hydrolysiert. Phosphonsäureester werden bevorzugt durch Erhitzen in hochkonzentrierten wäßrigen Säuren wie zum Beispiel konzentrierter Salzsäure gegebenenfalls unter Zusatz eines Alkohols oder durch Behandlung mit Trimethylsilylhalogenid in inerten Lösungsmitteln wie z.B. Acetonitril und anschließende Behandlung mit Wasser hydrolysiert.

Die Veresterung der Carbonsäure oder Phosphonsäure geschieht in an sich bekannter Weise mit dem entsprechenden Alkohol unter Säurekatalyse oder in Gegenwart eines aktivierten Säurederivats. Als aktivierte Säurederivate kommen zum Beispiel Säurechlorid, -imidazolid oder -anhydrid in Frage. Bei den Phosphonsäuren kann man die Veresterung durch Umsetzung mit Orthoestern gegebenenfalls unter Zusatz von Katalysatoren wie p-Toluolsulfonsäure erreichen.

Die Amidierung erfolgt an den freien Säuren oder an deren reaktiven Derivaten wie beispielsweise Säurechloriden, gemischten Anhydriden, Imidazoliden oder Aziden durch Umsetzung mit den entsprechenden Aminen bei Raumtemperatur.

Die Reduktion der Nitrogruppe zur Aminogruppe erfolgt katalytisch in polaren Lösungsmitteln bei Raumtemperatur oder erhöhter Temperatur. Als Katalysatoren sind Metalle wie Raney-Nickel oder Edelmetallkatalysatoren wie Palladium oder Platin gegebenenfalls auf Trägern geeignet. Statt Wasserstoff kann auch Ammoniumformiat in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Als Reduktionsmittel ist auch Eisen nutzbar. Die Reaktion wird dann in Gegenwart einer Säure wie z.B. Essigsäure oder Ammoniumchlorid gegebenenfallls unter Zusatz eines Lösungsmittels wie Wasser durchgeführt. Es kann vorteilhaft sein, vor der Reduktion die Estergruppe einzuführen. Bei Vorhandensein mehrerer Nitrogruppen im Molekül kann die gewünschte orthoständige Nitrogruppe auch selektiv in üblicher Weise mit Na₂S reduziert werden.

Wird eine Alkylierung einer Aminogruppe gewünscht, so kann nach üblichen Methoden beispielsweise mit Alkylhalogeniden oder nach der Mitsunobu Variante durch Umsetzung mit einem Alkohol in Gegenwart von Triphenylphosphin und Azodicarbonsäureester alkyliert werden, oder man unterwirft das Amin einer reduktiven Aminierung mit Aldehyden oder Ketonen gegebenenfalls nacheinander mit zwei verschiedenen Carbonylverbindungen, wobei man gemischte Derivate erhält (Literatur z.B. Verardo et al. Synthesis 1993, 121; Synthesis 1991, 447; Kawaguchi, Synthesis 1985, 701; Micovic et al. Synthesis 1991, 1043).

Die Acylierung einer Aminogruppe erfolgt in üblicher Weise beispielsweise mit einem Säurehalogenid oder Säureanhydrid gegebenenfalls in Gegenwart einer Base wie Dimethylaminopyridin in Lösungsmitteln wie Methylenchlorid, Tetrahydrofuran oder Pyridin oder nach der Schotten Baumann Variante in wäßriger Lösung bei schwach alkalischem pH-Wert.

Die Einführung der Cyanogruppe kann mit Hilfe der Sandmeyer-Reaktion erfolgen; beispielsweise kann man die aus den Aminoverbindungen mit Nitriten intermediär gebildeten Diazoniumsalze mit Alkalicyaniden in Gegenwart von Cu-I-cyanid umsetzen.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure oder mit Kaliumjodid umsetzt.

Wenn ein organischer Salpetrigsäureester benutzt wird, kann man die Halogene z.B. durch Zusatz von Methylenjodid oder Tetrabrommethan einführen in einem Lösungsmittel wie zum Beispiel Dimethylformamid.

Die Einführung von Fluor gelingt beispielsweise durch Balz Schiemann-Reaktion des Diazoniumtetrafluorborates.

Die Einführung einer NO₂-Gruppe gelingt durch eine Reihe von bekannten Nitrierungsmethoden. Beispielsweise kann mit Nitroniumtetrafluoroborat in inerten Lösungsmitteln wie halogenierten Kohlenwasserstoffen oder in Sulfolan, Eisessig oder Acetonitril nitriert werden. Möglich ist auch die Einführung z.B. durch Nitriersäure in konz. Schwefelsäure als Lösungsmittel bei Temperaturen zwischen 0°C und 30°C.

Die Einführung von Halogen gelingt durch bekannte Halogenierungsmethoden wie z.B. durch elektrophile aromatische Substitution.
Beispielsweise kann nach einem Verfahren mit Jod und Jodsäure von Wirth et al. [Liebigs Ann. Chem. 634, 84 (1960)] oder mit N-Jodsuccinimid in Lösungsmitteln wie Tetrahydrofuran, Dimethylformamid oder Trifluormethansulfonsäure jodiert werden.

Die Etherspaltung erfolgt nach den üblichen Methoden beispielsweise durch Reaktion mit Trimethylbromsilan gegebenenfalls unter Zusatz von Alkaliiodid in einem inerten Lösungsmittel wie Acetonitril bei einer Temperatur von 0°C bis zur Siedetemperatur des Lösungsmittels.

Die Einführung des Tetrazoles gelingt durch Umsetzung des entsprechenden Nitriles mit einem Azid wie z.B. Trimethylsilylazid, Stickstoffwasserstoffsäure oder Natriumazid gegebenenfalls unter Zusatz einer Protonenquelle wie z.B. Ammoniumchlorid oder Triethylammoniumchlorid in polaren Lösungsmitteln wie Dimethylformamid, Dimethylacetamid oder N-Methylpyrrolidon bei Temperaturen bis zum Siedepunkt des Lösungsmittels.

Die Überführung des Alkohols in das Halogenid gelingt mit Säurehalogeniden wie Thionylchlorid oder Phosphortribromid mit oder ohne Lösungsmittel. Als Lösungsmittel sind halogenierte Kohlenwasserstoffe, z.B. Methylenchlorid oder Ether möglich.

Die Isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung der Formel I mit der äquivalenten Menge oder einem Überschuß einer Base oder Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt oder analog zu bekannten Verbindungen, beispielsweise nach WO93/08173 oder hier beschriebenen Verfahren herstellbar.

Die nachfolgenden Beispiele sollen das erfindungsgemäße Verfahren erläutern:

### Herstellung der Ausgangsverbindungen

### 1. A)

2,05 g Mono-tert.butoxycarbonylethylendiamin werden mit 1,25 g (0,86ml) 4-Fluor-3-nitro-1-trifluormethylbenzol 30 min. auf 50°C Badtemperarur erwärmt. Die Masse verfestigt sich dabei. Anschließend wird über Kieselgel mit Methylenchlorid:Ethanol = 10:1 chromatographiert. Man erhält 2,2 g [1-N-Carbo t-butoxy 2 N-(2-nitro-4-trifluormethylphenyl)]ethylendiamin.

### B)

N-(2-Nitro-4-trifluormethyl-phenyl)ethylendiamin. 2,2 g 1-N-Carbo-t-butoxy-2-N-(2-nitro-4-trifluormethylphenyl)ethylendiamin werden in 60 ml Ethanol mit 60 ml 1-N-Salzsäure 2h auf 110°C erwärmt. Nach Einengen erhält man 1,77 g N-(2-Nitro-4-trifluormethyl-phenyl)ethylendiamin als Hydrochlorid.

### C)

[N-1-(Benzoyl)-N-2-(2-Nitro-4-trifluormethylphenyl)]ethylendiamin. 570 mg N-(2-Nitro-4-trifluormethylphenyl)ethylendiamin hydrochlorid werden in 15 ml Methylenchlorid zunächst mit 424 mg Triethylamin und dann mit 295 mg Benzoylchlorid versetzt. Nach 2h Rühren bei Raumtemperatur wird zweimal mit Wasser extrahiert. Die organische Phase wird getrocknet, filtriert und eingeengt. Man erhält 690 mg [N-1-(Benzoyl)-N-2-(2-Nitro-4-trifluormethylphenyl)]ethylendiamin.

### Analog wird hergestellt

[N-1-(Methansulfonyl)-N-2-(-2-Nitro-4-trifluormethylphenyl)]ethylendiamin.
N-1-(4-Chlor-benzoylamino)-N-2-(2-nitro-4-trifluormethylphenyl) ethylendiamin.
N-1-Nicotinoylamino)-N-2-(2-nitro-4-trifluormethylphenyl) ethylendiamin
N-1-(Phenylsulfonylamino)-N-2-(2-nitro-4-trifluormethylphenyl) ethylendiamin

### 2. A) 1 -(2-Nitro-4-trifluormethylphenylamino)-2-methoxyethan

3,75 g 2-Methoxyethylamin und 10,5 g 4-Fluor-3-nitro-1-trifluormethylbenzol werden in 200 ml Wasser mit 10 g Natriumcarbonat 2h auf 100°C Badtemperatur erwärmt. Beim Abkühlen fällt das Produkt aus, das sich durch Absaugen isolieren läßt (9,8 g). Die wäßrige Mutterlauge wird mit 4-N-Salzsäure ausgesäuert und dreimal mit je 100 ml Essigester extrahiert. Die gesammelten organischen Extrakte werden getrocknet, filtriert und eingeengt. Man erhält nochmals 2,7 g. Gesamtausbeute 12,5 g 1-(2-Nitro-4-trifluormethylphenyl)amino-2-methoxyethan.

### 3. A)

1,1 g 2,4-Difluornitrobenzol werden mit 2,8 g Aminomethanphosphonsäurediethylester 2h auf 40°C erwärmt. Anschließend wird über Kieselgel mit Methylenchlorid:Ethanol = 10:1 chromatographiert. Man erhält 1,6 g N-(2-Nitro-5-fluorphenyl)aminomethanphosphonsäurediethylester.

### Analog wird hergestellt.

N-(2-Nitro-4-fluorphenyl)-aminomethanphosphonsäurediethylester
1-[N-(2-Nitro-4-fluorphenyl)amino]-ethanphosphonsäurediethylester
1-[N-(2-Nitro-5-fluorphenyl)-amino]-ethanphosphonsäurediethylester
1-(2-Nitro-4-trifluormethylphenylamino)-4-methoxypropanphosphonsäurediethylester.

### B)

331 mg N-Benzophenoniminylmethanphosphonsäurediethylester werden mit 40 mg Aliquat 336 und mit 209 mg 2-Methoxy-1-bromethan vorgelegt und bei 0°C mit 280 mg gepulvertem Kaliumhydroxyd versetzt; anschließend wird bei Raumtemperatur 3,5h gerührt. Der Ansatz wird mit Methylenchlorid und 180 mg Kieselgel versetzt, kurz gerührt und abgesaugt. Das eingeengte Filtrat wird über Kieselgel mit Cyclohexan:Essigester = 1:1 chromatographiert. Man erhält 180 mg 2-(N-Benzophenoniminyl)-4-methoxy-propanphosphonsäurediethylester.

### C)

2,0 g 2-(N-Benzophenoniminyl)-4-methoxy-propanphosphonsäurediethylester werden in 30 ml 1-N-HCl und 30 ml Diethylether 3h bei Raumtemperatur gerührt. Es wird die organische Phase abgetrennt und die Wasserphase nochmals mit Diethylether extrahiert. Die organische Phase enthält Benzophenon und wird verworfen. Die wäßrige Phase wird zur Trockene eingeengt, in 15 ml gesättigter Kochsalzlösung aufgenommen, mit Na₂CO₃ neutralisiert und dreimal mit 50 ml Methylenchlorid extrahiert. Die organische Phase wird getrocknet, filtriert und eingeengt und ergibt 800 mg 2-Amino-4-methoxy-propanphosphonsäurediethylester.

### 4. A)

790 mg N-(2-Nitro-5-fluorphenyl) aminomethanphosphonsäurediethylester werden in 50 ml Ethanol mit 2,5 g Raney Nickel versetzt und unter Wasserstoffnormaldruck bei Raumtemperatur 2h hydriert. Nach Absaugen vom Katalysator wird eingeengt. Man erhält 660 mg N-(2-Amino-5-fluorphenyl) aminomethanphosphonsäurediethylester.

### Analog werden hergestellt

N-(2-Amino-4-fluorphenyl) aminomethanphosphonsäurediethylester,
1-[(2-Amino-4-trifluormethylphenyl) amino*]-4*-methoxypropanphosphonsäurediethylester
1-[N-(2-Amino-4-fluorphenyl)amino]-ethanphosphonsäurediethylester
1-[N-(2-Amino-5-fluorphenyl)-amino]-ethanphosphonsäurediethylester
1-(2-Amino-4-trifluormethylphenyl) amino-2-methoxyethan
N-1-(Methansulfonyl)-N-2-(-2-amino-4-trifluormethylphenyl) ethylendiamin
N-1-(benzoylamino)-N-2-(2-amino-4-trifluormethylphenyl) ethylendiamin
N-1-(4-Chlor-benzoylamino)-N-2-(2-amino-4-trifluormethylphenyl) ethylendiamin.
N-1-Nicotinoylamino)-N-2-(2-amino-4-trifluormethylphenyl) ethylendiamin
N-1-(Phenylsulfonylamino)-N-2-(2-amino-4-trifluormethylphenyl) ethylendiamin

### Beispiel 1

### (7-Fluor-1,2,3,4-tetrahydro-2,3-dioxochinoxalin-1-yl)-methanphosphonsäurediethylester

660 mg N(-2 Amino-5-fluorphenyl)-aminomethanphosphonsäurediethylester werden in 70 ml absoluten Tetrahydrofuran mit 509 mg Triethylamin vorgelegt. Zu dieser Lösung wird langsam eine Lösung von 685 mg Oxalsäureethylesterchlorid in 30 ml Tetrahydrofuran getropft. Der Ansatz wird 4h bei Raumtemperatur gerührt. Nach Absaugen der ausgefallenen Salze wird das Filtrat eingeengt, in einem Gemisch von 23 ml Ethanol und 23 ml 1-N-Salzsäure für 2h bei 110°C Badtemperatur gekocht. Es wird zur Trockene eingeengt und der Rückstand über Kieselgel mit Methylenchlorid:Ethanol = 10:1 chromatographiert. Man erhält 561 mg (7-Fluor-1,2,3,4-tetrahydro-2,3-dioxochinoxalin-1-yl) methanphosphonsäurediethylester.

### Analog werden hergestellt:

(6-Fluor-1,2,3,4-tetrahydro-2,3-dioxo chinoxalin-1-yl)methanphosphonsäurediethylester
1-[(7-Fluor-1,2,3,4-tetrahydro-2,3-dioxo chinoxalin-1-yl)]-ethanphosphonsäurediethylester
1-[(6-Fluor-1,2,3,4-tetrahydro-2,3-dioxo chinoxalin-1-yl)] ethanphosphonsäurediethylester

### Grundsätzlich analog werden hergestellt:

6-Trifluormethyl-1-(1-methoxyeth-2-yl)-1,2,3,4-tetrahydro-2,3-dioxo-chinoxalin.
6-Trifluormethyl-1-(1-N-benzoylaminoeth-2-yl)-1,2,3,4-tetrahydro-2,3-dioxo-chinoxalin
6-Trifluormethyl-1-(1-N-methansulfonylaminoeth-2-yl)-1,2,3,4-tetrahydro-2,3-dioxo-chinoxalin
1-(6-Trifluormethyl-1,2,3,4-tetrahydro-2,3-dioxo-chinoxalin-1-yl)-3-methoxy-propanphosphonsäurediethylester.
6-Trifluormethyl-1-(1-N-phenylsulfonylaminoeth-2-yl)-1,2,3,4-tetrahydro-2,3-dioxo-chinoxalin
6-Trifluormethyl-1-(1-N-nicotinoylamino-2-yl)-1,2,3,4-tetrahydro-2,3-dioxo-chinoxalin
6-Trifluormethyl-1-(1-N-4-chlor-benzoylaminoeth-2-yl)-1,2,3,4-tetrahydro-2,3-dioxo-chinoxalin

### Beispiel 2

1 g 6-Trifluormethyl-1-(1-methoxyeth-2-yl)-1,2,3,4-tetrahydro-2,3-dioxo-chinoxalin werden in 20 ml absoluten Acetonitril mit 3,15 ml Trimethylbromsilan und 1,4 g Natriumjodid versetzt und 1h auf 80°C Badtemperatur erwärmt. Nach Zugabe von 25 ml Wasser wird mit Essigester extrahiert. Die organische Phase wird abgetrennt, eingeengt und über Kieselgel mit Toluol:Eisessig:Wasser = 10:10:1 chromatographiert. Nach Einengen der entsprechenden Fraktionen und Ausrühren mit Ethanol erhält man 330 mg 6-Trifluormethyl-1-(1-hydroxyeth-2-yl)-1,2,3,4-tetrahydro-2,3-dioxo-chinoxalin.

### Beispiel 3

615 mg (7-Fluor-1,2,3,4-tetrahydro-2,3-dioxo-chinoxalin-1yl-) methanphosphonsäurediethylester werden in 60 ml Methylenchlorid mit 743 mg Nitroniumtetrafluorborat versetzt. Der Ansatz wird 2h bei Raumtemperatur gerührt. Es wird mit 50 ml Wasser versetzt und nach Abtrennen der organischen Phase dreimal mit Methylenchlorid extrahiert. Die gesammelte organische Phase wird getrocknet, filtriert und eingeengt. Der Rückstand wird über Kieselgel mit Methylenchlorid:Ethanol = 10:1 chromatographiert. Man erhält 350 mg (6-Nitro-7-Fluor-1,2,3,4-tetrahydro-2,3-dioxochinoxalin-1-yl)-methanphosphonsäurediethylester.

### Grundsätzlich analog werden hergestellt:

(6-Fluor-7-nitro-1,2,3,4-tetrahydro-2,3-dioxochinoxalin-1-yl) methanphosphonsäurediethylester.
1-[(6-Fluor-7-Nitro-1,2,3,4-tetrahydro-2,3-dioxochinoxalin-1-yl)] ethanphosphonsäurediethylester.
1-[(7-Fluor-6-Nitro-1,2,3,4-tetrahydro-2,3-dioxochinoxalin-1-yl)] ethanphosphonsäurediethylester.
1-[(6-Trifluormethyl-7-Nitro-1,2,3,4-tetrahydro-2,3-dioxochinoxalin-1-yl)] ethanphosphonsäurediethylester
[(6-Trifluormethyl-7-Nitro-1,2,3,4-tetrahydro-2,3-dioxochinoxalin-1-yl)] methanphosphonsäurediethylester.

### Beispiel 4 140 mg (6-Nitro-7-fluor-1,2,3,4-tetrahydro-2,3-dioxochinoxalin-1-yl) methanphosphonsäurediethylester werden mit 129 mg Morpholin 1,5h auf 120°C Badtemperatur erwärmt. Nach Einengen im Vakuum wird der Rückstand über Kieselgel mit Toluol:Eisessig:Wasser = 10:10:1 chromatographiert. Nach Einengen der entsprechenden Fraktionen erhält man 300 mg (7-Morpholino-6-nitro-1,2,3,4-tetrahydro-2,3-dioxochinoxalin-1-yl) methanphosphonsäurediethylester.

### Analog wird hergestellt:

[6-(N-Imidazolyl)-7-nitro-1,2,3,4-tetrahydro-2,3-dioxochinoxalin-1-yl] methanphosphonsäurediethylester
1-[6-(N-Imidazolyl)-7-nitro-1,2,3,4-tetrahydro-2,3-dioxochinoxalin-1-yl] ethanphosphonsäurediethylester
1-[7-(N-Imidazolyl)-6-nitro-1,2,3,4-tetrahydro-2,3-dioxochinoxalin-1-yl] ethanphosphonsäurediethylester
(6-Morpholino-7-nitro-1,2,3,4-tetrahydro-2,3-dioxochinoxalin-1-yl) methanphosphonsäurediethylester
1-[(6-Morpholino-7-nitro-1,2,3,4-tetrahydro-2,3-dioxochinoxalin-1-yl)] ethanphosphonsäurediethylester
1-[(7-Morpholino-6-nitro-1,2,3,4-tetrahydro-2,3-dioxochinoxalin-1-yl)] ethanphosphonsäurediethylester
1-[(7-2-(Methoxyethylamino-6-nitro-1,2,3,4-tetrahydro-2,3-dioxochinoxalin-1-yl)] methanphosphonsäurediethylester
1-[(7-N-Methylpiperazinyl-6-nitro-1,2,3,4-tetrahydro-2,3-dioxochinoxalin-1-yl)] methanphosphonsäurediethylester
1-[7-(4-Methylimidazol-1-yl)-6-nitro-1,2,3,4-tetrahydro-2,3-dioxochinoxalin-1-yl] methanphosphonsäurediethylester
1-[7-(2-Methylimidazol-1-yl)-6-nitro-1,2,3,4-tetrahydro-2,3-dioxochinoxalin-1-yl] methanphosphonsäurediethylester
1-[7-(2,4-Dimethylimidazol-1-yl)-6-nitro-1,2,3,4-tetrahydro-2,3-dioxochinoxalin-1-yl] methanphosphonsäurediethylester
(7-Thiomorpholino-6-nitro-1,2,3,4-tetrahydro-2,3-dioxochinoxalin-1-yl) methanphosphonsäurediethylester
(7-N,N-Dipropylamino-6-nitro-1,2,3,4-tetrahydro-2,3-dioxochinoxalin-1-yl) methanphosphonsäurediethylester
(7-N,N-Dipropylamino-6-nitro-1,2,3,4-tetrahydro-2,3-dioxochinoxalin-1-yl) ethanphosphonsäurediethylester
(7-N-Methyl-N-propylamino-6-nitro-1,2,3,4-tetrahydro-2,3-dioxochinoxalin-1-yl) methanphosphonsäurediethylester
[7-(1,2,4-Triazol-1-yl)-6-nitro-1,2,3,4-tetrahydro-2,3-chinoxalin-1-yl]methanphosphonsäurediethylester

### Beispiel 5

375 mg (7-Fluor-6-nitro-1,2,3,4-tetrahydro-2,3-dioxochinoxalin-1-yl)methanphosphonsäurediethylester werden zu einer Lösung gegeben, die aus 200 mg Trifluorethanol und 60 mg Natriumhydrid (80%ig) in 20 ml absoluten Tetrahydrofuran hergestellt wurde. Nach der Zugabe wird 4,5h auf 70°C Badtemperatur geheizt. Es wird eingeengt, in 50 ml Wasser aufgenommen, mit 1N-Salzsäure sauer gestellt und dreimal mit Essigester extrahiert.
Die organische Phase wird getrocknet, filtriert und eingeengt. Der Rückstand wird über Kieselgel mit Toluol:Eisessig:Wasser = 10:10:1 chromatographiert. Nach Einengen der entsprechenden Fraktionen und Ausrühren mit Ethanol erhält man in Form des Rückstandes 19 mg (6-Nitro-7-trifluorethoxy-1,2,3,4-tetrahydro-2,3-dioxo-chinoxalin-1-yl)methanphosphonsäurediethylester.

### Beispiel 6

259 mg (6-Nitro-7-morpholino-1,2,3,4-tetrahydro-2,3-dioxo-chinoxalin-1-yl)-methanphosphonsäurediethylester werden in 10 ml absoluten Acetonitril mit 628 mg Trimethylbromsilan versetzt und 1h bei Raumtemperatur gerührt. Es wird wenig Wasser zugegeben und zur Trockene eingeengt. Der Rückstand wird über silanisiertes Kieselgel mit Methanol als Elutionsmittel chromatographiert. Man erhält 60 mg (6-Nitro-7-morpholino-1,2,3,4-tetrahydro-2,3-dioxo-chinoxalin-1-yl)methanphosphonsäure.

### Analog werden hergestellt:

(6-Morpholino-7-nitro-1,2,3,4-tetrahydro-2,3-dioxochinoxalin-1-yl) methanphosphonsäure
1-[(6-Morpholino-7-nitro-1,2,3,4-tetrahydro-2,3-dioxochinoxalin-1-yl)] ethanphosphonsäure
1-[(7-Morpholino-6-nitro-1,2,3,4-tetrahydro-2,3-dioxochinoxalin-1-yl)] ethanphosphonsäure
1-[(7-(2-Methoxyethylamino-6-nitro-1,2,3,4-tetrahydro-2,3-dioxochinoxalin-1-yl)] methanphosphonsäure
1-[(7-N-Methylpiperazinyl-6-nitro-1,2,3,4-tetrahydro-2,3-dioxochinoxalin-1-yl)] methanphosphonsäure
(7-Thiomorpholino-7-nitro-1,2,3,4-tetrahydro-2,3-dioxochinoxalin-1-yl) methanphosphonsäure
[(6-Trifluormethyl-7-morpholino-chinoxalin-2,3-dion)-1-yl]-methanphosphonsäure;
   Schmelzpunkt > 300°C, ¹H-NMR in DMSO: 7,9 s, 1H; 7,4 s, 1H; 4,6 d, 11Hz, 2H: 3,7 t 5Hz, 2H; 2,9 t, 5Hz, 2H.

### In analoger Weise werden hergestellt:

[(6-Trifluormethyl-7-[piperidin-1-yl]chinoxalin-2,3-dion)-1-yl]-methanphosphonsäure; Schmelzpkt > 300°C;
[(6-Trifluormethyl-7-[2,6-dimethyl-(morpholin-1-yl)]chinoxalin-2,3-dion)-1yl]-methanphosphonsäure;
[(6-Trifluormethyl-7-(hexahydroazepin-1-yl)chinoxalin-2,3-dion)-1-yl]-methanphosphonsäure;
[(6-Trifluormethyl-7-[(4-phenylpiperazin-1-yl)chinoxalin-2,3-dion)-1-yl]-methanphosphonsäure;
1-[(6-Trifluormethyl-7-[morpholin-1-yl]chinoxalin-2,3-dion)-1-yl]-ethanphosphonsäure;

### Beispiel 7

250 mg [(6-(N-Imidazolyl)-7-nitro-1,2,3,4-tetrahydro-2,3-dioxochinoxalin-1-yl] methanphosphonsäurediethylester werden in 3 ml konzentrierter Salzsäure 2,5h auf 110°C Badtemperatur erwärmt. Nach Einengen wird in Wasser aufgenommen und das ausgefallene Produkt abgesaugt. Man erhält 100 mg [6-(N-Imidazolyl)-7-nitro-1,2,3,4-tetrahydro-2,3-dioxochinoxalin-1-yl] methanphosphonsäure.

### Analog werden hergestellt:

(6-Nitro-7-fluor-1,2,3,4-tetrahydro-2,3-dioxo-chinoxalin-1-yl) methanphosphonsäure
(7-Nitro-6-fluor-1,2,3,4-tetrahydro-2,3-dioxochinoxalin-1-yl) methanphosphonsäure
1-[6-(N-Imidazolyl)-7-nitro-1,2,3,4-tetrahydro-2,3-dioxochinoxalin-1-yl] ethanphosphonsäure
1-[7-(N-Imidazolyl)-6-nitro-1,2,3,4-tetrahydro-2,3-dioxochinoxalin-1-yl] ethanphosphonsäure
1-[(6-Fluor-7-Nitro-1,2,3,4-tetrahydro-2,3-dioxochinoxalin-1-yl)] ethanphosphonsäure
1-[(7-Fluor-6-Nitro-1,2,3,4-tetrahydro-2,3-dioxochinoxalin-1-yl)] ethanphosphonsäure
1-[7-(2-Methylimidazol-1-yl)-6-nitro-1,2,3,4-tetrahydro-2,3-dioxochinoxalin-1-yl] methanphosphonsäure
1-[7-(4-Methylimidazol-1-yl)-6-nitro-1,2,3,4-tetrahydro-2,3-dioxochinoxalin-1-yl] methanphosphonsäure
1-[7-(2,4-Dimethylimidazol-1-yl)-6-nitro-1,2,3,4-tetrahydro-2,3-dioxochinoxalin-1-yl] methanphosphonsäure
(7-NN-Dipropylamino-6-nitro-1,2,3,4-tetrahydro-2,3-dioxochinoxalin-1-yl) methanphosphonsäure
(7-N,N-Dipropylamino-6-nitro-1,2,3,4-tetrahydro-2,3-dioxochinoxalin-1-yl) methanphosphonsäure
(7-N-Methyl-N-propylamino-6-nitro-1,2,3,4-tetrahydro-2,3-dioxochinoxalin-1-yl) methanphosphonsäure
1-[(6-Trifluormethyl-7-imidazolyl-chinoxalin-2,3-dion)-1-yl]ethanphosphonsäure
1-[(6-Trifluormethyl-7-(-4-methylimidazol-1-yl-chinoxalin-2,3-dion)-1yl] - methanphosphonsäure
[6-Trifluormethyl-7-amino-1,2,3,4-tetrahydro-2,3-dioxo-chinoxalin-1yl]-methanphosphonsäure
1-[6-Trifluormethyl-7-amino-1,2,3,4-tetrahydro-2,3-dioxo-chinoxalin-1yl]-ethanphosphonsäure
1-[(6-Trifluormethyl-7-[piperidin-1-yl]chinoxalin-2,3-dion)-1yl]-ethanphosphonsäure
1-[6-Trifluormethyl-7-propylamino-1,2,3,4-tetrahydro-2,3-dioxo-chinoxalin-1-yl]-ethan-1-phosphonsäure
1-[6-Trifluormethyl-7-hexylamino-1,2,3,4-tetrahydro-2,3-dioxo-chinoxalin-1-yl]-ethan-1-phosphonsäure
[(6-Trifluormethyl-7-[hex-1-ylamino]-chinoxalin-2,3-dion)-1yl]-methanphosphonsäure
[(6-Trifluormethyl-7-[pent-1-ylamino]-chinoxalin-2,3-dion)-1yl]-methanphosphonsäure
[(6-Trifluormethyl-7-[hex-2-ylamino]-chinoxalin-2,3-dion)-1yl]-methanphosphonsäure
[7-(1,2,4-Triazol-1-yl)-6-nitro-1,2,3,4-tetrahydro-2,3-chinoxalin-1-yl]methanphosphonsäure.

### Beispiel 8

### (6 Trifluormethyl-7-morpholinochinoxalin-2,3-dion-1-yl)-methanphosphonsäurediethylester

### A

3,3 g (30 mmol) Aminomethanphosphonsäure werden in 120 ml Wasser und 120 ml Ethanol zusammen mit 3,37 g (31,8 mmol) Soda vorgelegt und mit 7,8 g (97 %ig, 30 mmol) 3-Trifluormethyl-4,6-dichlornitrobenzol versetzt und 4 h bei 120°C Badtemperatur am Rückfluß gerührt. Nach Abziehen des Ethanol am Rotationsverdampfer wird dreimal mit 100 ml Essigester extrahiert. Die organische Phase wird mit wenig Wasser gewaschen. Sie enthält Ausgangsmaterial und wird verworfen. Die gesammelte wäßrige Phase wird mit 4N-Salzsäure sauer auf pH1 gestellt und dreimal mit je 100 ml Essigester extrahiert. Die organische Phase wird mit Wasser gewaschen, getrocknet, filtriert und eingeengt. Man erhält 6,85 g (68 % d. Th.) N-(2-Nitro-4-trifluormethyl-5-chlor-phenyl)-aminomethanphosphonsäure vom Schmelzpunkt 207,3°C.

### Analog wird hergestellt:

1-[(2-Nitro-4-trifluormethyl-5-chlor-phenyl)-amino]ethanphosphonsäure.

### B, C

6,85 g (20,5 mmol) N-(2-Nitro-4-trifluormethyl-5-chlor-phenyl)-aminomethanphosphonsäure werden in 20 ml Morpholin 3,5 h bei 120°C Badtemperatur gerührt. Es wird am Rotationsverdampfer zur Trockene eingeengt und der Rückstand mit 100 ml Orthoameisensäuretriethylester und 779 mg (4.1 mmol) p-Toluolsulfonsäure 3.8 h auf 150°C Badtemperatur erwärmt. Nach Einengen am Rotationsverdampfer zur Trockene wird in 100 ml Wasser aufgenommen, mit Kochsalz versetzt und dreimal mit je 100 ml Essigester extrahiert. Die gesammelte Essigesterphase wird mit verdünnter Kochsalzlösung gewaschen, getrocknet, filtriert und eingeengt. Ausbeute 10,6g (> 100 % d.Th., enthält noch Morpholinhydrochlorid) an N-(2-Nitro-4-trifluormethyl-5-morpholinophenyl)-amino methanphosphonsäurediethylester.

### D

10,6 g (∼20 mmol) N-(2-Nitro-4-trifluormethyl-5-morpholino-phenyl)-aminomethanphosphonsäurediethylester werden in 250 ml Ethanol mit 4,5g Palladium auf Kohle (10 %ig) unter Wasserstoffnormaldruck bei Raumtemperatur 3,5h hydriert. Nach Absaugen des Katalysators über Kieselgur und Einengen des Filtrats erhält man 9,2 g (>100 % d.Th., enthält noch Morpholinhydrochlorid) an N-(2-Amino-4-trifluormethyl-5-morpholino-phenyl)-aminomethanphosphonsäurediethylester.

### In analoger Weise über B, C, D werden hergestellt:

N-(2-Amino-4-trifluormethyl-5-piperidino-phenyl)-aminomethanphosphonsäurediethylester
N-(2-Amino-4-trifluormethyl-5-(-2,6-dimethylmorpholino)-phenyl)-aminomethanphosphonsäurediethylester
N-(2-Amino-4-trifluormethyl-5-hexahydroazepino-phenyl)-aminomethanphosphonsäurediethylester
N-(2-Amino-4-trifluormethyl-5-phenylpiperazinyl-phenyl)-aminomethanphosphonsäurediethylester
1-[(2-Amino-4-trifluormethyl-5-morpholinophenyl)-amino]ethanphosphonsäurediethylester
N[(2-Amino-4-trifluormethyl-5-hex-1-ylaminophenyl)-amino]methanphosphorsäurediethyl-ester
N[(2-Amino-4-trifluormethyl-5-pent-1-ylaminophenyl)-amino]methanphosphorsäurediethyl-ester
N[(2-Amino-4-trifluormethyl-5-hex-2-ylaminophenyl)-amino]methanphosphorsäurediethyl-ester

### E

1,26 g (2,7 mmol) N-(2-Amino-4-trifluormethyl-5-morpholino-phenyl)-aminomethanphosphonsäurediethylester werden in 60 ml absolutem Tetrahydrofuran zusammen mit 0,79 ml (5,7 mmol) Triethylamin vorgelegt. Die Mischung wird tropfenweise mit einer Lösung von 0,63 ml (5,7 mmol) Oxalsäureetylesterchlorid in 20 ml Tetrahydrofuran versetzt. Anschließend wird 3 h bei Raumtemperatur gerührt. Der Ansatz wird abgesaugt und das Filtrat eingeengt. Der Rückstand des eingeengten Filtrats wird in 20 ml Ethanol und 20 ml 1-N Salzsäure aufgenommen und 2h bei 110°C Badtemperatur gerührt. Nach Abziehen des Ethanols am Vakuum wird mit Wasser auf 30 ml verdünnt und dreimal mit je 30 ml Essigester ausgeschüttelt. Die gesammelte Essigesterphase wird einmal mit 30 ml Wasser gewaschen, getrocknet, filtriert und eingeengt. Man erhält 1,13 g (89,7 % d.Th.) [(6 Trifluormethyl-7-morpholinochinoxalin-2,3-dion)-1yl]methanphosphonsäurediethylester vom Schmelzpunkt 192,6°C.

### In analoger Weise werden hergestellt:

[(6-Trifluormethyl-7-[piperidin-1-yl]chinoxalin-2,3-dion)-1yl]-methanphosphonsäurediethylester;
[(6-Trifluormethyl-7-[2,6-dimethyl-(morpholin-1-yl)]chinoxalin-2,3-dion)-1yl]-methanphosphonsäurediethylester;
[(6-Trifluormethyl-7-(hexahydroazepin-1-yl)chinoxalin-2,3-dion)-1yl]-methanphosphonsäure-diethylester;
[(6-Trifluormethyl-7-[(4-phenylpiperazin-1-yl)chinoxalin-2,3-dion)-1yl]-methanphosphonsäurediethylester;
1-[(6-Trifluormethyl-7-[morpholin-1-yl]-chinoxalin-2,3-dion)-1yl]-ethanphosphonsäurediethylester;
[(6-Trifluormethyl-7-[hex-1-ylamino]-chinoxalin-2,3-dion)-1yl]-methanphosphonsäurediethylester;
[(6-Trifluormethyl-7-[pent-1-ylamino]-chinoxalin-2,3-dion)-1yl]-methanphosphonsäurediethylester;
[(6-Trifluormethyl-7-[hex-2-ylamino]-chinoxalin-2,3-dion)-1yl]-methanphosphonsäurediethylester.

### Beispiel 9

### 1-(6 Trifluormethyl-7-imidazolylchinoxalin-2,3-dion-1-yl)-ethanphosphonsäurediethylester

### A

1,67 g (5 mmol) 1-[(2-Nitro-4-trifluormethyl-5-chlor-phenyl)amino]ethanphosphonsäure (nach Beispiel 8, A) werden zusammen mit 1,70 g Imidazol 4 h bei 160°C Badtemperatur gerührt. Es wird in 100 ml Wasser aufgenommen und zweimal mit Ionenaustauscher IR 120 (stark saure Form, Amberlite, 20 - 50 mesh) gerührt und abgesaugt. Anschließend wird am Rotationsverdampfer zur Trockene eingeengt und der Rückstand mit 24 ml Orthoameisensäuretriethylester und 156 mg (0,82 mmol) p-Toluolsulfonsäure 10 h auf 150°C Badtemperatur erwärmt. Nach Einengen am Rotationsverdampfer zur Trockene wird über Kieselgel mit Methylenchlorid: Ethanol = 10: 1 chromatographiert. Man erhält 222 mg (17 % d.Th.) an 1-[(2-Nitro-4-trifluormethyl-5-imidazolylphenyl)-amino]ethanphosphonsäurediethylester.

### In analoger Weise werden hergestellt:

1-[(2-Nitro-4-trifluormethyl-5(4-methylimidazol-1-yl)-phenyl)-amino]methanphosphonsäurediethylester.

### B

572 mg (1,4 mmol) 1-[(2-Nitro-4-trifluormethyl-5-imidazolyl-phenyl)-amino]ethanphosphonsäurediethylester werden in 60 ml Ethanol mit 300 mg Palladium auf Kohle (10 %ig) unter Wasserstoffnormaldruck bei Raumtemperatur 1,5h hydriert. Nach Absaugen des Katalysators über Kieselgur und Einengen des Filtrats erhält man 531 mg (99,6% d.Th.) an 1-[(2-Amino-4-trifluormethyl-5-imidazolylphenyl)-amino]ethanphosphonsäurediethyl-ester.

### In analoger Weise werden hergestellt:

1-[(2-Amino-4-trifluormethyl-5(4-methylimidazol-1-yl)-phenyl)-amino]methanphosphonsäurediethylester.

### C

531 mg (1,4 mmol) 1-[(2-Amino-4-trifluormethyl-5-imidazolyl-phenyl)-amino]ethanphosphonsäurediethylester werden in 35 ml absolutem Tetrahydrofuran zusammen mit 0,4 ml (2,9 mmol) Triethylamin vorgelegt. Die Mischung wird tropfenweise mit einer Lösung von 0,32 ml (2,9 mmol) Oxalsäureethylesterchlorid in 10 ml Tetrahydrofuran versetzt. Anschließend wird 3 h bei Raumtemperatur gerührt. Der Ansatz wird abgesaugt und das Filtrat eingeengt. Der Rückstand des eingeengten Filtrats wird in 15 ml Ethanol und 15 ml 1-N Salzsäure aufgenommen und 2h bei 110°C Badtemperatur gerührt. Nach Abziehen des Ethanols am Vakuum wird mit Wasser auf 30 ml verdünnt und dreimal mit je 25 ml Essigester ausgeschüttelt. Die gesammelte Essigesterphase wird einmal mit 30 ml Wasser gewaschen, getrocknet, filtriert und eingeengt. Man erhält 1-[(6-Trifluormethyl-7-imidazolylchinoxalin-2,3-dion)-1yl]ethanphosphonsäurediethylester.

### In analoger Weise werden hergestellt:

1-[(6-Trifluormethyl-7-(-4-methylimidazol-1-yl)-chinoxalin-2,3-dion)-1yl]methanphosphon-säurediethylester.

### Beispiel 10

### [6-Trifluormethyl-7-amino-1,2,3,4-tetrahydro-2,3-dioxo-chinoxalin-1-yl]-methanphosphonsäurediethylester

600 mg [6-Trifluormethyl-7-nitro-1,2,3,4-tetrahydro-2,3-dioxo-chinoxalin-1yl]methanphosphonsäureester werden in 30ml Ethanol gelöst und zusammen mit 100 mg Pd/C **(10%)** 1h bei Wasserstoffnormaldruck und Raumtemperatur hydriert. Nach Absaugen vom Katalysator wird der Katalysator nochmals mit Ethanol ausgekocht, das gesammelte Filtrat eingeengt und man erhält 590 mg [6-Trifluormethyl-7-amino-1,2,3,4-tetrahydro-2,3-dioxo-chinoxalin-1yl]-methanphosphonsäurediethylester.

### Analog werden hergestellt:

[6-Trifluormethyl-7-amino-1,2,3,4-tetrahydro-2,3-dioxo-chinoxalin-1yl]-methanphosphonsäurediethylester.

### Beispiel 11

### 6-Trifluormethyl-7-jod-1-(1-methoxyeth-2-yl)-1,2,3,4-tetrahydro-2,3-dioxochinoxalin

90 mg 6-Trifluormethyl-7-amino-1-(1-methoxyeth-2-yl)-1,2,3,4-tetrahydro-2,3-dioxochinoxalin werden in 8 ml ethanolischer Salzsäure aufgenommen und eingeengt und das Hydrochlorid in 6 ml Dimethylformamid und 3 ml Methylenjodid aufgenommen und bei 80°C Badtemperatur mit 0,08 ml i-Amylnitrit versetzt. Nach 3h Rühren bei dieser Temperatur wird der Ansatz am Vakuum eingeengt. Man erhält 105 mg 6-Trifluormethyl-7-jod-1-(1-methoxyeth-2-yl)-1,2,3,4-tetrahydro-2,3-dioxo-chinoxalin.

### In analoger Weise werden hergestellt:

[6-Trifluormethyl-7-jod-1,2,3,4-tetrahydro-2,3-dioxo-chinoxalin-1-yl]methanphosphonsäurediethylester.

### Beispiel 12

### 1-[(6-Trifluormethyl-7-[piperidin-1-yl]chinoxalin-2,3-dion)-1yl]-ethanphosphonsäure-diethylester

Bei 0°C wird zu einer Aufschlemmung von 100 mg [(6-Trifluormethyl-7-amino-1-yl]chinoxalin-2,3-dion)-1yl]-methanphosphonsäure-diethylester und 30 mg Natiumborhydridtablettenstücken in 3 ml Tetrahydrofuran eine Lösung von 0,15 ml einer 25 %igen wäßrigen Lösung von Glutardialdehyd und 0,45 ml 3-M Schwefelsäure in 3 ml Tetrahydrofuran:Methanol = 2:3 getropft. Nach Abklingen der Reaktion werden noch einmal 30 mgNatiumborhydridtablettenstücken nachgeworfen und anschließend 1h bei Raumtemperatur gerührt. Dann wird mit Natronlauge neutral gestellt und mit Essigester ausgeschüttelt. Die Essigesterphase wird getrocknet, filtriert und eingeengt. Nach Chromatographie über Kieselgel mit Toluol:Eisessig:Wasser = 10:10:1 erhält man 60 mg 1-[(6-Trifluormethyl-7-[piperidin-1-yl]chinoxalin-2,3-dion)-1yl]-ethanphosphonsäurediethylester.

### In analoger Weise werden hergestellt:

1-[(6-Trifluormethyl-7-[piperidin-1-yl]chinoxalin-2,3-dion)-1yl]-essigsäurethylester;

### Beispiel 13

### 1-[(6-Trifluormethyl-7-propylamino-]chinoxalin-2,3-dion)-1yl]-ethanphosphonsäurediethylester;

Bei 4°C wird zu einer Aufschlemmung von 80 mg [(6-Trifluormethyl-7-amino-1-yl]chinoxalin-2,3-dion)-1yl]-methanphosphonsäure-diethylester in 3 ml Tetrahydrofuran eine Lösung von 0,02 ml destilliertem Propanal und 0,2 ml 3-M Schwefelsäure in 2 ml Tetrahydrofuran getropft. Zu dieser gerührten Mischung werden 20 mg Natiumborhydridtablettenstücken gegeben. Nach Abklingen der Reaktion werden noch einmal 10 mg Natiumborhydridtablettenstücken nachgeworfen und anschließend 1h bei Raumtemperatur gerührt. Dann wird mit Natronlauge neutral gestellt und mit Essigester ausgeschüttelt. Die Essigesterphase wird getrocknet, filtriert und eingeengt. Nach Chromatographie über Kieselgel mit Toluol:Eisessig: Wasser = 10:10:1 erhält man 40 mg 1-[(6-Trifluormethyl-7-propylamino-chinoxalin-2,3-dion)-1yl]-ethanphosphonsäure-diethylester.

## Patentansprüche

1. Verbindungen der Formel I 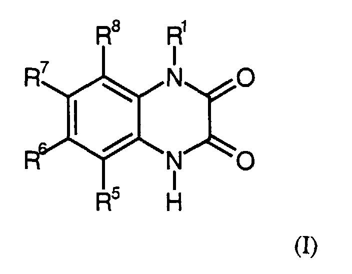 worin
R¹ -(CH₂)ₙ-CR²H-(CH₂)ₘ-Z und
R⁵, R⁶, R⁷ und R⁸ gleich oder verschieden sind und Wasserstoff C₁₋₆-Alkyl, CF₃, Nitro, Halogen, NR⁹R¹⁰, Cyano, SOₚR¹¹, SO₂NR¹²R¹³, SO₃H, SO₃C₁₋₆-Alkyl oder OR¹⁴ bedeuten,
wobei
R² Wasserstoff oder -(CH₂)_{q}-R³,
R³ Wasserstoff, Hydroxy, C₁₋₆-Alkoxy oder NR¹⁵R¹⁶,
n, m und q jeweils 0, 1, 2 oder 3
Z POXY oder OPOXY,
R¹¹ H, C₁₋₆-Alkyl, Phenyl,
p 0, 1 oder 2
R¹², R¹³ Wasserstoff oder C₁₋₄-Alkyl,
R¹⁴ H oder gegebenenfalls 1-3 fach mit Halogen substituiertes C₁₋₆-Alkyl,
X und Y gleich oder verschieden sind und Hydroxy, C₁₋₆-Alkoxy, C₁₋₄-Alkyl oder NR¹⁸R¹⁹ bedeuten,
R⁹ und R¹⁰ gleich oder verschieden sind und Wasserstoff, Phenyl oder C₁₋₆-Alkyl, das gegebenenfalls mit C₁₋₄-Alkoxy oder einer gegebenenfalls mit C₁₋₄-Alkyl mono- oder di-substituierter Aminogruppe substituiert sein kann, oder gemeinsam mit dem Stickstoffatom einen 5 - 7gliedrigen gesättigten Heterocyclus bilden, der ein weiteres N-, S- oder O-Atom enthalten und substituiert sein kann oder einen ungesättigten 5-gliedrigen Heterocyclus bilden, der 1 - 3 N-Atome enthalten und substituiert sein kann,
R¹⁵ und R¹⁶, R¹⁸ und R¹⁹ gleich oder verschieden sind und Wasserstoff, C₁₋₄-Alkyl, Phenyl oder gemeinsam mit dem Stickstoffatom einen 5 - 7gliedrigen gesättigten Heterocyclus bilden, der ein weiteres Sauerstoff-, Schwefel- oder Stickstoffatom enthalten und substituiert sein kann oder einen ungesättigten 5gliedrigen Heterocyclus bilden, der 1 - 3 N-Atome enthalten und substituiert sein kann,
sowie deren Isomeren und Salze, die in Position 5 - 8 zweifach substituiert sind und,
falls R¹ Methanphosphonsäure und R⁶ CF₃ oder NO₂ und R⁷ Imidazol ist, können R⁵ und R⁸ nicht gleichzeitig Wasserstoff sein.

2. (6-Nitro-7-trifluorethoxy-1,2,3,4-tetrahydro-2,3-dioxo-chinoxalin-1-yl)methanphosphonsäurediethylester
(6-Nitro-7-morpholino-1,2,3,4-tetrahydro-2,3-dioxo-chinoxalin-1-yl)methanphosphon-säure
1-[(7-Morpholino-6-nitro-1,2,3,4-tetrahydro-2,3-dioxochinoxalin-1-yl)]ethanphosphon-säure
1-[(7-(2-Methoxyethylamino)-6-nitro-1,2,3,4-tetrahydro-2,3-dioxochinoxalin-1-yl)]methanphosphonsäure
[(6-Trifluormethyl-7-morpholinochinoxalin-2,3-dion)-1yl)methanphosphonsäure
[(6-Trifluormethyl-7-[piperidin-1-yl]chinoxalin-2,3-dion)-1yl]methanphosphonsäure
[(6-Trifluormethyl-7-[2,6-dimethyl-(morpholin-1-yl)]chinoxalin-2,3-dion)-1-yl]methan-phosphonsäure
[(6-Trifluormethyl-7-[azepin-1-yl]chinoxalin-2,3-dion)-1-yl]methanphosphonsäure
[(6-Trifluormethyl-7-[4-phenylpiperazin-1-yl]chinoxalin-2,3-dion)-1-yl]methanphosphonsäure
1-[(6-Trifluormethyl-7-[morpholin-1-yl]-chinoxalin-2,3-dion-)-1-yl]ethanphosphonsäure
1-[(6-Trifluormethyl-7-imidazolylchinoxalin-2,3-dion)-1-yl]ethanphosphonsäure
1-[(6-Trifluormethyl-7-(4-methylimidazol-1-yl)-chinoxalin-2,3-dion)-1yl]methanphosphonsäure
[6-Trifluormethyl-7-amino-1,2,3,4-tetrahydro-2,3-dioxo-chinoxalin-1yl]methanphosphonsäure
1-[7-(2-Methylimidazol-1-yl)-6-nitro-1,2,3,4-tetrahydro-2,3-dioxochinoxalin-1-yl]methanphosphonsäure
1-[7-(4-Methylimidazol-1-yl)-6-nitro-1,2,3,4-tetrahydro-2,3-dioxochinoxalin-1-yl]methanphosphonsäure
1-[7-(2,4-Dimethylimidazol-1-yl)-6-nitro-1,2,3,4-tetrahydro-2,3-dioxochinoxalin-1-yl]methanphosphonsäure.

3. Verbindungen nach Anspruch 1, die in Position 6 und 7 substituiert sind.

4. Verbindungen nach Anspruch 1, worin R⁵-R⁸ NR⁹R¹⁰ und CF₃ bedeuten.

5. Arzneimittel enthaltend eine Verbindung nach Anspruch 1 und einen oder mehrere pharmazeutisch geeignete Träger- und Hilfsstoffe.

6. Verfahren zur Herstellung der Verbindungen der Formel I, dadurch gekennzeichnet, daß man
a) eine Verbindung der Formel II 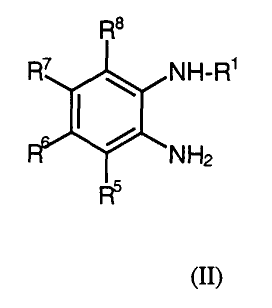 worin R¹ bis R⁸ die obige Bedeutung haben, mit Oxalsäure oder reaktiven Oxalsäurederivaten cyclisiert oder
b) eine Verbindung der Formel III 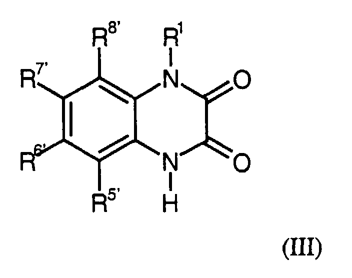 worin R¹ die obige Bedeutung hat und einer der Substituenten R^{5'}, R^{6'}, R^{7'} oder R^{8'} eine Fluchtgruppe darstellt, nucleophil substituiert und gewünschtenfalls anschließend die Estergruppe verseift oder die Säuregruppe verestert oder amidiert oder die Nitrogruppe reduziert zur Aminogruppe oder die Aminogruppe alkyliert oder acyliert oder die Aminogruppe gegen Halogen oder Cyano austauscht oder eine Nitrogruppe oder Halogen einführt oder eine Etherspaltung vornimmt oder den Alkohol in Halogenid überführt oder dieses Halogen nukleophil substituiert oder ein Nitril in das Tetrazol überführt oder die Isomeren trennt oder die Salze bildet.

7. Verbindungen nach Anspruch 1, die in 5-, 6-, 7- und/oder 8-Stellung mit C₁₋₆-Alkyl, CF₃, NO₂, Halogen, SOₚR¹¹, SO₂NR¹²R¹³ oder NR⁹R¹⁰ substituiert sind.

8. Verwendung einer Verbindung nach Anspruch 1 zur Herstellung eines Arzneimittels zur Behandlung einer durch Hyperaktivität excitatorischer Aminosäuren ausgelösten Erkrankung.

9. Verwendung einer Verbindung nach Anspruch 1 zur Herstellung eines Arzneimittels zur Behandlung neurologischer und psychiatrischer Erkrankungen.

10. Verwendung nach Anspruch 9 zur funktionellen und präventiven Behandlung neurodegenerativer Erkrankungen.

11. Verwendung nach Anspruch 10 zur Behandlung von Stroke.

## Claims

1. Compounds of the formula I 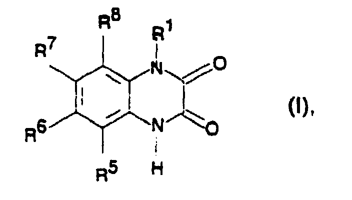 in which
R¹ denotes -(CH₂)ₙ-CR²H-(CH₂)ₘ-Z and
R⁵, R⁶, R⁷ and R⁸ are identical or different and denote hydrogen C₁₋₆-alkyl, CF₃, nitro, halogen, NR⁹R¹⁰, cyano, SOₚR¹¹, SO₂NR¹²R¹³, SO₃H SO₃C₁₋₆-alkyl or OR¹⁴, where
R² denotes hydrogen or -(CH₂)_{q}-R³,
R³ denotes hydrogen, hydroxyl, C₁₋₆-alkoxy or NR¹⁵R¹⁶,
n, m and q each denote 0,1, 2 or 3
Z denotes POXY or OPOXY,
R¹¹ denotes H, C₁₋₆-alkyl, phenyl,
p denotes 0, 1 or 2
R¹², R¹³ denote hydrogen or C₁₋₄-alkyl,
R¹⁴ denotes H or C₁₋₆-alkyl which is optionally substituted 1-3 times by halogen,
X and Y are identical or different and denote hydroxyl,
C₁₋₆-alkoxy, C₁₋₄-alkyl or NR¹⁸R¹⁹,
R⁹ and R¹⁰ are identical or different and denote hydrogen, phenyl or C₁₋₆-alkyl which may optionally be substituted by C₁₋₄-alkoxy or an amino group which is optionally mono- or di-substituted by C₁₋₄-alkyl, or form together with the nitrogen atom a 5-7-membered saturated heterocycle which may contain another N, S or O atom and may be substituted, or form an unsaturated 5-membered heterocycle which may contain 1-3 N atoms and may be substituted, R¹⁵ and R¹⁶, R¹⁸ and R¹⁹ are identical or different and denote hydrogen, C₁₋₄-alkyl, phenyl or form together with the nitrogen atom a 5-7-membered saturated heterocycle which may contain another oxygen, sulphur or nitrogen atom and may be substituted, or form an unsaturated 5-membered heterocycle which may contain 1-3 N atoms and may be substituted, and their isomers and salts which are disubstituted in position 5-8, and if R¹ is methanephosphonic acid and R⁶ is CF₃ or NO₂ and R⁷ is imidazole, it is not possible for R⁵ and R⁸ both to be hydrogen.

2. Diethyl (6-nitro-7-trifluoroethoxy-1,2,3,4-tetrahydro-2,3-dioxoquinoxalin-1-yl)methanephosphonate
(6-nitro-7-morpholino-1,2,3,4-tetrahydro-2,3-dioxoquinoxalin-1-yl)methanephosphonic acid
1-[(7-morpholino-6-nitro-1,2,3,4-tetrahydro-2,3-dioxoquinoxalin-1-yl)]ethanephosphonic acid
1-[(7-(2-methoxyethylamino)-6-nitro-1,2,3,4-tetrahydro-2,3-dioxoquinoxalin-1-yl)]methanephosphonic acid
[(6-trifluoromethyl-7-morpholinoquinoxaline-2,3-dion)-1-yl)methanephosphonic acid
[(6-trifluoromethyl-7-[piperidin-1-yl]quinoxaline-2,3-dion)-1-yl]methanephosphonic acid
[(6-trifluoromethyl-7-[2,6-dimethyl-(morpholin-1-yl)]quinoxaline-2,3-dion)-1-yl]methanephosphonic acid
[(6-trifluoromethyl-7-[azepin-1-yl]quinoxalin-2,3-dion)-1-yl]methanephosphonic acid
[(6-trifluoromethyl-7-[4-phenylpiperazin-1-yl]quinoxaline-2,3-dion)-1-yl]methanephosphonic acid
1-[(6-trifluoromethyl-7-[morpholin-1-yl]quinoxaline-2,3-dion)-1-yl]ethanephosphonic acid
1-[(6-trifluoromethyl-7-imidazolylquinoxaline-2,3-dion)-1-yl]ethanephosphonic acid
1-[(6-trifluoromethyl-7-(4-methylimidazol-1-yl)quinoxaline-2,3-dion)-1-yl]methanephosphonic acid
[6-trifluoromethyl-7-amino-1,2,3,4-tetrahydro-2,3-dioxoquinoxalin-1-yl]methanephosphonic acid
1-[7-(2-methylimidazol-1-yl)-6-nitro-1,2,3,4-tetrahydro-2,3-dioxoquinoxalin-1-yl]methanephosphonic acid
1-[7-(4-methylimidazol-1-yl)-6-nitro-1,2,3,4-tetrahydro-2,3-dioxoquinoxalin-1-yl]methanephosphonic acid
1-[7[(2,4-dimethylimidazol-1-yl)-6-nitro-1,2,3,4-tetrahydro-2,3-dioxoquinoxalin-1-yl]methanephosphonic acid.

3. Compounds according to Claim 1, which are substituted in position 6 and 7.

4. Compounds according to Claim 1, in which R⁵-R⁸ denote NR⁹R¹⁰ and CF₃.

5. Pharmaceuticals containing a compound according to Claim 1 and one or more pharmaceutically suitable carriers and excipients.

6. Process for the preparation of compounds of the formula I, characterized by
a) cyclization of a compound of the formula II 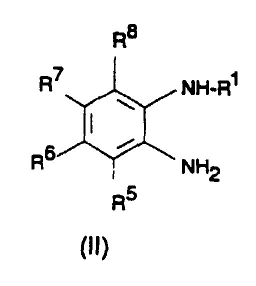 in which R¹ to R⁸ have the above meaning, with oxalic acid or reactive oxalic acid derivatives or
b) nucleophilic substitution of a compound of the formula III 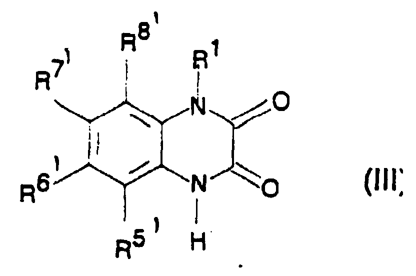 in which R¹ has the above meaning, and one of the substituents R^{5'}, R^{6'}, R^{7'} or R^{8'} represents a leaving group, and, if required, subsequent hydrolysis of the ester group or esterification or amidation of the acid group or a reduction of the nitro group to an amino group or alkylation or acylation of the amino group or replacement of the amino group by halogen or cyano or introduction of a nitro group or halogen or performance of an ether cleavage or conversion of the alcohol into a halide or nucleophilic substitution of this halogen or conversion of a nitrile into a tetrazole or separation of the isomers or formation of salts.

7. Compounds according to Claim 1, which are substituted in position 5, 6, 7 and/or 8 by C₁₋₆-alkyl, CF₃, NO₂, halogen, SOₚR¹¹, SO₂NR¹²R¹³ or NR⁹R¹⁰.

8. Use of a compound according to Claim 1 for producing a pharmaceutical for treating a disorder caused by hyperactivity of excitatory amino acids.

9. Use of a compound according to Claim 1 for producing a pharmaceutical for treating neurological and psychiatric disorders.

10. Use according to Claim 9 for the functional and preventive treatment of neurodegenerative disorders.

11. Use according to Claim 10 for treating stroke.

## Revendications

1. Composés de formule I 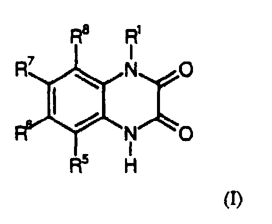 dans laquelle
R¹ signifie -(CH₂)ₙ-CR²H-(CH₂)ₘ-Z et
R⁵, R⁶, R⁷ et R⁸ sont identiques ou différents et signifient un hydrogène, un alkyle en C₁-C₆, CF₃, un nitro, un halogène, NR⁹R¹⁰, un cyano, SOₚR¹¹, SO₂NR¹²R¹³, SO₃H, un SO₃alkyle en C₁-C₆ ou OR¹⁴,
R² étant un hydrogène ou -(CH₂)_{q}-R³,
R³ étant un hydrogène, un hydroxyle, un alcoxy en C₁-C₆ ou NR¹⁵R¹⁶,
n, m et q étant chaque fois 0, 1, 2 ou 3,
Z étant POXY ou OPOXY,
R¹¹ étant H, un alkyle en C₁-C₆, un phényle,
p étant 0, 1 ou 2,
R¹², R¹³ étant un hydrogène ou un alkyle en C₁-C₄,
R¹⁴ étant H ou un alkyle en C₁-C₆ le cas échéant 1 à 3 fois substitué par un halogène,
X et Y étant identiques ou différents et signifiant un hydroxyle, un alcoxy en C₁-C₆, un alkyle en C₁-C₄ ou NR¹⁸R¹⁹,
R⁹ et R¹⁰ sont identiques ou différents et sont un hydrogène, un phényle ou un alkyle en C₁-C₆ qui, le cas échéant, peut être substitué par un alcoxy en C₁-C₄ ou par un groupement amino le cas échéant mono-ou disubstitué par un alkyle en C₁-C₄, ou forment conjointement avec l'atome d'azote un hétérocycle saturé de 5 à 7 membres qui peut contenir un autre atome de N, de S ou de O et peut être substitué ou forment un hétérocyle à cinq membres insaturé qui peut contenir 1 à 3 atomes de N et peut être substitué,
R¹⁵ et R¹⁶, R¹⁸ et R¹⁹ sont identiques ou différents et sont un hydrogène, un alkyle en C₁-C₄, un phényle ou forment conjointement avec l'atome d'azote un hétérocyle saturé de 5 à 7 membres qui peut contenir un autre atome d'oxygène, de soufre ou d'azote et peut être substitué ou forment un hétérocyle à 5 membres insaturé qui peut contenir de 1 à 3 atomes de N et peut être substitué,
ainsi que leurs isomères et sels qui sont deux fois substitués en position 5 à 8 et, au cas ou R¹ est un acide méthanephosphonique et R⁶ est CF₃ ou NO₂ et R⁷ est un imidazole, R⁵ et R⁸ ne peuvent pas être simultanément un hydrogène.

2. Ester diéthylique d'acide de (6-nitro-7-trifluoroéthoxy-1,2,3,4-tétrahydro-2,3-dioxoquinoxalin-1-yl)méthanephosphonique.
Acide (6-nitro-7-morpholino-1,2,3,4-tétrahydro-2,3-dioxoquinoxalin-1-yl)méthanephosphonique
Acide 1-[(7-morpholino-6-nitro-1,2,3,4-tétrahydro-2,3-dioxoquinoxalin-1-yl)]éthanephosphonique
Acide 1-[(7-(2-méthoxyéthylamino)-6-nitro-1,2,3,4-tétrahydro-2,3-dioxoquinoxalin-1-yl)]méthanephosphonique
Acide [(6-trifluorométhyl-7-morpholinoquinoxalin-2,3-dion)-1-yl)méthanephosphonique
Acide [(6-trifluorométhyl-7-[pipéridin-1-yl]quinoxalin-2,3-dion)-1-yl]méthanephosphonique
Acide [(6-trifluorométhyl-7-[2,6-diméthyl(morpholin-1-yl)]quinoxalin-2,3-dion)-1-yl]méthanephosphonique
Acide [(6-trifluorométhyl-7-[azépin-1-yl]quinoxalin-2,3-dion)-1-yl]méthanephosphonique
Acide [(6-trifluorométhyl-7-[4-phénylpipérazin-1-yl]quinoxalin-2,3-dion)-1-yl]méthanephosphonique
Acide 1-[(6-trifluorométhyl-7-[morpholin-1-yl]quinoxalin-2,3-dion)-1-yl]éthanephosphonique
Acide 1-[(6-trifluorométhyl-7-imidazolylquinoxalin-2,3-dion)-1-yl]éthanephosphonique
Acide 1-[(6-trifluorométhyl-7-(4-méthylimidazol-1-yl)-quinoxalin-2,3-dion)-1-yl]méthanephosphonique
Acide [6-trifluorométhyl-7-amino-1,2,3,4-tétrahydro-2,3-dioxoquinoxalin-1-yl]méthanephosphonique
Acide 1-[7-(2-méthylimidazol-1-yl)-6-nitro-1,2,3,4-tétrahydro-2,3-dioxoquinoxalin-1-yl]méthanephosphonique
Acide 1-[7-(4-méthylimidazol-1-yl)-6-nitro-1,2,3,4-tétrahydro-2,3-dioxoquinoxalin-1-yl]méthanephosphonique
Acide 1-[7-(2,4-diméthylimidazol-1-yl)-6-nitro-1,2,3,4-tétrahydro-2,3-dioxoquinoxalin-1-yl]méthanephosphonique.

3. Composés suivant la revendication 1, qui sont substitués en position 6 et 7.

4. Composés suivant la revendication 1, dans lesquels R⁵-R⁸ signifient NR⁹R¹⁰ et CF₃.

5. Médicament contenant un composé suivant la revendication 1 et un ou plusieurs supports et auxiliaires pharmaceutiquement appropriés.

6. Procédé de préparation des composés de formule I, caractérisé en ce que
a) on cyclise avec de l'acide oxalique ou des dérivés réactifs d'acide oxalique un composé de formule II 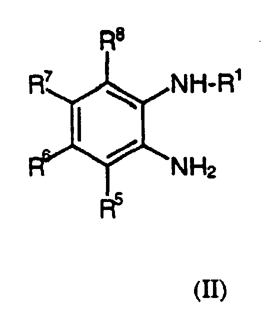 dans laquelle R¹ à R⁸ ont la signification susmentionnée ou
b) on substitue de manière nucléophile un composé de formule III 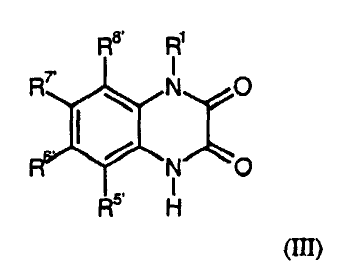 dans laquelle R¹ a la signification susmentionnée et un des substituants R^{5'}, R^{6'}, R^{7'} ou R^{8'} représente un groupement linéaire, puis, si on le souhaite on saponifie le groupement ester ou on estérifie ou on transforme en amide le groupement acide ou on réduit le groupement nitro en un groupement amino ou on alkyle ou acyle le groupement amino ou on échange le groupement amino contre un halogène ou un cyano ou on introduit un groupement nitro ou un halogène ou on entreprend une séparation de l'éther ou on transforme l'alcool en halogénure ou on substitue cet halogène de manière nucléophile ou on transforme un nitrile en le tétrazole ou on sépare les isomères ou on forme les sels.

7. Composés suivant la revendication 1, qui sont substitués en position 5, 6, 7 et/ou 8 avec un alkyle en C₁-C₆, CF₃, NO₂, un halogène, SOₚR¹¹, SO₂NR¹²R¹³ ou NR⁹R¹⁰.

8. Utilisation d'un composé selon la revendication 1 pour la préparation d'un médicament destiné au traitement d'un trouble déclenché par l'hyperactivité d'acides aminés excitateurs.

9. Utilisation d'un composé selon la revendication 1 pour la préparation d'un médicament destiné au traitement de troubles neurologiques et psychiatriques.

10. Utilisation selon la revendication 9, pour le traitement fonctionnel et préventif de troubles neurodégénératifs.

11. Utilisation selon la revendication 10, pour le traitement d'attaques.
